# EUROPÄISCHE PATENTSCHRIFT

(11) **EP 0 778 259 B1**
(45) Veröffentlichungstag und Bekanntmachung des Hinweises auf die Patenterteilung: **24.01.2001**
(21) Anmeldenummer: 96119145.9
(22) Anmeldetag: 29.11.1996
(51) Int. Cl.: C07C 209/60

(54) **Verfahren zur Herstellung von Aminen aus Olefinen an Zeolithen des Typs SSZ-37**
Process for the preparation of amines from olefines using SSZ-37 zeolithes
Procédé pour la préparation d'amines à partir d'oléfines utilisant des zéolithes du type SSZ-37

(30) Priorität: 08.12.1995 DE 19545876
(43) Veröffentlichungstag der Anmeldung: 11.06.1997
(73) Patentinhaber: BASF AKTIENGESELLSCHAFT, 67056 Ludwigshafen (DE)
(72) Erfinder: Eller, Karsten, Dr., 67059 Ludwigshafen (DE); Kummer, Rudolf, Dr., 67227 Frankenthal (DE); Stops, Peter, 67122 Altrip (DE)

(56) Entgegenhaltungen:
- EP-A- 0 431 451
- EP-A- 0 587 424
- WO-A-94/00233
- US-A- 5 254 514
- CORMA A ET AL: "INFLUENCE OF THE METHOD OF DEALUMINATION OF Y ZEOLITES ON ITS BEHAVIOUR FOR CRACKING N-HEPTANE AND VACUUM GAS-OIL" STUDIES IN SURFACE SCIENCE AND CATALYSIS, Bd. 37, Nr. 15, 1. Januar 1988, Seiten 495-503, XP000197448

## Beschreibung

Die vorliegende Erfindung betrifft ein Verfahren zur Herstellung von Aminen durch Umsetzung von Ammoniak oder primären oder sekundären Aminen mit Olefinen bei erhöhten Temperaturen und Drücken in Gegenwart von Zeolithen des Typs SSZ-37.

Eine Übersicht über die Methoden zur Aminierung von Olefinen wird in "Functionalisation of Alkenes: Catalytic Amination of Monoolefins", J.J. Brunet et al. J. Mol. Catal., 49 (1989), Seiten 235 bis 259 gegeben.

Grundsätzlich gibt es zwei Katalysemechanismen. Das Olefin wird über einen Metallkomplex koordiniert. Diese aktivierte Spezies kann von dem nucleophilen Amin angegriffen werden und ein höher aminiertes Produkt bilden. Das Amin kann an Säurezentren oder an Metallzentren (via Metallamide) chemisorbiert werden und so aktiviert mit dem Olefin umgesetzt werden.

Gut geeignete Katalysatoren sind Zeolithe. Sie zeichnen sich durch eine hohe Anzahl an katalytisch aktiven Zentren aus, kombiniert mit einer großen Oberfläche. Die beschriebenen Zeolithe unterscheiden sich im Typ und in der Nachbehandlung (z.B. thermische Behandlung, Dealuminierung, Säurebehandlung, Metallioneneintausch, etc.). Beispiele hierfür finden sich in US-A-4 375 002, US-A-4 536 602, EP-A-305 564, EP-A-101 921, DE-A-42 06 992.

Aus EP-A-133 938, EP-A-431 451 und EP-A-132 736 sind Verfahren bekannt, bei denen Bor-, Gallium-, Alumino- und Eisensilikatzeolithe für die Herstellung von Aminen aus Olefinen verwendet werden und auf die Möglichkeit der Dotierung dieser Zeolithe mit Alkali-, Erdalkali- und Übergangsmetallen hingewiesen wird.

Aus CA-A-2 092 964 ist ein Verfahren zur Herstellung von Aminen aus Olefinen bekannt, bei dem BETA-Zeolithe, die als kristalline Aluminosilikate bestimmter Zusammensetzung mit einer Porengröße von mehr als 5 Ä definiert sind, eingesetzt werden. Bevorzugt werden metall- oder halogenmodifizierte Beta-Zeolithe eingesetzt.

Aus EP-A 587 424 ist ein Verfahren zur Herstellung von Aminen aus Olefinen bekannt, bei dem als Katalysatoren Y-Zeolithe eingesetzt werden.

Alle Verfahren zur Synthese von Aminen aus Olefinen an diesen Katalysatoren zeichnen sich durch eine geringe Amin-Ausbeute oder geringe Raum-Zeit-Ausbeute aus, oder führen zu einer raschen Desaktivierung der Katalysatoren.

Der vorliegenden Erfindung lag daher die Aufgabe zugrunde, diesen Nachteilen abzuhelfen.

Demgemäß wurde ein neues und verbessertes Verfahren zur Herstellung von Aminen der allgemeinen Formel I 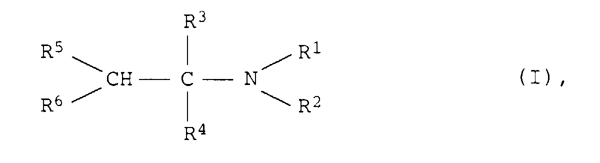 in der
- R¹,R²,R³,R⁴,R⁵,R⁶: Wasserstoff, C₁- bis C₈-Alkyl, C₂- bis C₈-Alkenyl, C₂H, Propargyl, C₂- bis C₈-Cycloalkyl, C₅- bis C₁₀-Alkyl-cycloalkyl oder C₂- bis C₁₀-Cycloalkyl-alkyl,
- R¹ und R²: gemeinsam eine gesättigte oder ungesättigte C₃- bis C₉-Alkylendikette und
- R³ und R⁵: gemeinsam eine C₂- bis C₈-Alkylendikette
bedeuten, durch Umsetzung von Olefinen der allgemeinen Formel II 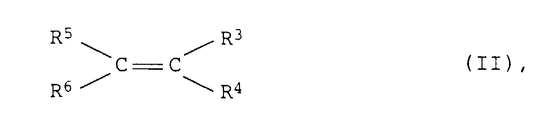 in der R³, R⁴, R⁵ und R⁶ die oben genannten Bedeutungen haben, mit Ammoniak oder primären oder sekundären Aminen der allgemeinen Formel III 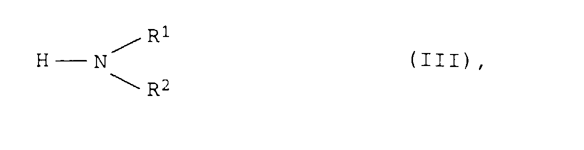 in der R¹ und R² die oben genannten Bedeutungen haben, bei Temperaturen von 200 bis 350°C und Drücken von 100 bis 300 bar in Gegenwart eines Heterogenkatalysators gefunden, welches dadurch gekennzeichnet ist, daß man als Heterogenkatalysator Zeolithe des Typs SSZ-37 einsetzt.

Das erfindungsgemäße Verfahren kann wie folgt durchgeführt werden:

Das Olefin II und Ammoniak oder das primäre oder sekundären Amin III kann bei Temperaturen von 200 bis 350°C, bevorzugt 220 bis 330°C, besonders bevorzugt 230 bis 320°C und Drücken von 100 bis 300 bar, bevorzugt 120 bis 300 bar, besonders bevorzugt 140 bis 290 bar in Gegenwart von Zeolithen des Typs SSZ-37 als Katalysator z.B. in einem Druck-Reaktor umsetzt werden, und bevorzugt das erhaltene Amin abgetrennt und die nichtumgesetzten Einsatzstoffe zurückführt werden.

Das vorliegende Verfahren zeichnet sich durch eine sehr gute Ausbeute bei hoher Selektivität und bei hoher Raum-Zeit-Ausbeute aus. Zudem ist die Desaktivierung des Katalysators zurückgedrängt worden.

Das erfindungsgemäße Verfahren zeichnet sich dadurch aus, daß bereits mit niedrigem Ammoniak- bzw. Amin- Überschuß eine hohe Selektivität an gewünschtem Reaktionsprodukt erzielt und die Dimerisierung und/oder Oligomerisierung des eingesetzten Olefins vermieden wird.

Eine Ausführungsform dieses Verfahrens besteht darin, daß man Ammoniak und/oder Amine III zusammen mit dem Olefin II im molaren Verhältnis von 1:1 bis 5:1 gemischt einem Festbettreaktor zuführt und bei einem Druck von 100 bis 300 bar und einer Temperatur von 200 bis 350°C in der Gasphase oder im überkritischen Zustand umsetzt.

Aus dem Reaktionsaustrag kann das gewünschte Produkt mit Hilfe bekannter Methoden, beispielsweise Destillation oder Extraktion, erhalten und nötigenfalls mittels weiterer Trennoperationen auf die gewünschte Reinheit gebracht werden. Die nichtumgesetzten Eingangsstoffe werden in der Regel bevorzugt in den Reaktor zurückgeführt.

Man kann einfach oder mehrfach ungesättigte Olefine II, insbesondere solche mit 2 bis 10 C-Atomen bzw. deren Mischungen und Polyolefine als Ausgangsstoffe verwenden. Wegen der geringer ausgeprägten Polymerisationsneigung eignen sich Monoolefine besser als Di- und Polyolefine, doch können diese mit Hilfe höherer Ammoniak- bzw. Aminüberschüsse ebenso selektiv umgesetzt werden. Die Lage des Gleichgewichts und damit der Umsatz zum gewünschten Amin ist sehr stark vom gewählten Reaktionsdruck abhängig. Hoher Druck begünstig das Additionsprodukt, doch stellt im allgemeinen aus technischen und wirtschaftlichen Gründen der Druckbereich bis 300 bar das Optimum dar. Die Selektivität der Reaktion wird - neben Größen wie Ammoniak-/Amin-Überschuß und Katalysator - in hohem Maß durch die Temperatur beeinflußt. Zwar nimmt die Reaktionsgeschwindigkeit der Additionsreaktion mit steigender Temperatur stark zu, doch werden konkurrierende Crack- und Rekombinationsreaktionen des Olefins gleichzeitig gefördert. Zudem ist eine Temperaturerhöhung aus thermodynamischer Sicht nicht vorteilhaft. Die Lage des Temperaturoptimums bezüglich Umsatz und Selektivität ist von der Konstitution des Olefins, des eingesetzten Amins und des Katalysators abhängig und liegt meist im Bereich von 200 bis 350°C.

Als Katalysatoren für die Aminierung von Olefinen eignen sich Zeolithe des Typs SSZ-37, die beispielsweise aus US-A-5 254 514 oder WO-A-94/00233 bekannt sind.

Die erfindungsgemäßen Zeolithe SSZ-37 können als solche verformt werden, oder aber auch mit einem Bindemittel im Verhältnis 98:2 bis 40:60 Gew.-% zu Strängen oder Tabletten. Als Bindemittel eignen sich verschiedene Aluminiumoxide, bevorzugt Boehmit, amorphe Aluminosilikate mit einem SiO₂/Al₂O₃-Verhältnis von 25:75 bis 95:5, Siliciumdioxid, bevorzugt hochdisperses SiO₂, Gemische aus hochdispersem SiO₂ und hochdispersem Al₂O₃, hochdisperses TiO₂ sowie Tone. Nach der Verformung werden die Extrudate oder Preßlinge zweckmäßig bei 110°C/16 h getrocknet und bei 200 bis 500°C/2 bis 16 h calciniert, wobei die Calcinierung auch direkt im Aminierungsreaktor erfolgen kann.

Zur Erhöhung der Selektivität, der Standzeit und der Anzahl der möglichen Regenerierungen kann man verschiedene Modifizierungen an den erfindungsgemäßen Zeolithkatalysatoren SSZ-37 vornehmen.

Eine Modifizierung der Katalysatoren besteht darin, daß man die unverformten oder die verformten Zeolithe mit Alkalimetallen wie Na und K, Erdalkalimetallen wie Ca, Mg, Erdmetallen wie Tl, Übergangsmetallen wie z.B. Ti, Zr, Mn, Fe, Mo, Cu, Zn, Cr, Edelmetallen und/oder seltenen Erdmetallen wie z.B. La, Ce oder Y ionenaustauschen bzw. dotieren kann.

Eine vorteilhafte Ausführungsform besteht darin, daß man die verformten erfindungsgemäßen Zeolithe SSZ-37 in einem Strömungsrohr vorlegt und bei 20 bis 100°C z.B. ein Halogenid, ein Acetat, ein Oxalat, ein Citrat oder ein Nitrat der oben beschriebenen Metalle in gelöster Form darüberleitet. Ein derartiger Ionenaustausch kann z.B. an der Wasserstoff-, Ammonium- und Alkaliform der erfindungsgemäßen Zeolithe SSZ-37 vorgenommen werden.

Eine weitere Möglichkeit der Metallaufbringung auf die erfindungsgemäßen Zeolithe SSZ-37 besteht darin, daß man das Material z.B. mit einem Halogenid, einem Acetat, einem Oxalat, einem Citrat, einem Nitrat oder einem Oxid der oben beschriebenen Metalle in wäßriger oder alkoholischer Lösung imprägniert.

Sowohl an einen Ionenaustausch als auch an eine Imprägnierung kann man eine Trocknung, wahlweise eine abermalige Calcination anschließen. Bei metalldotierten Zeolithen des Typs SSZ-37 kann eine Nachbehandlung mit Wasserstoff und/oder mit Wasserdampf günstig sein.

Eine weitere Möglichkeit der Modifizierung besteht darin, daß man die erfindungsgemäßen Zeolithe SSZ-37 - verformt oder unverformt - einer Behandlung mit Säuren, wie Salzsäure (HCl), Flußsäure (HF), Schwefelsäure (H₂SO₄), Oxalsäure (HO₂C-CO₂H), Phosphorsäure (H₃PO₄) oder deren Gemischen unterwirft.

Eine besondere Ausführungsform besteht darin, daß man die erfindungsgemäßen Zeolithe SSZ-37 vor der Verformung mit einer der genannten Säuren 0,001 n bis 2 n, bevorzugt 0,05 bis 0,5 n 1 bis 100 Stunden unter Rückfluß behandelt. Nach Abfiltrieren und Auswaschen wird in der Regel bei 100 bis 160°C getrocknet und bei 200 bis 600°C calciniert. Eine weitere besondere Ausführungsform liegt in einer Säure-Behandlung der erfindungsgemäßen Zeolithe SSZ-37 nach ihrer Verformung mit Bindemittel. Hierbei wird der erfindungsgemäße Zeolith in der Regel 1 bis 3 Stunden zwischen 60 und 80°C mit einer 3 bis 25%igen, insbesondere mit einer 12 bis 20%igen Säure behandelt, anschließend ausgewaschen, bei 100 bis 160°C getrocknet und bei 200 bis 600°C calciniert. Auch hier kann die Calcinierung wieder direkt im Aminierungsreaktor erfolgen.

Eine andere Möglichkeit der Modifizierung ist gegeben durch einen Austausch mit Ammonsalzen, z.B. mit NH₄Cl oder mit Mono-, Di- oder Polyaminen. Hierbei wird der mit Bindemittel verformte Zeolith in der Regel bei 60 bis 80°C mit 10 bis 25%iger, bevorzugt 20%iger NH₄Cl-Lösung 2 h kontinuierlich in gewichtsmäßiger Zeolith/Ammonchlorid-Lösung von 1:15 ausgetauscht und danach bei 100 bis 120°C getrocknet.

Eine weitere Modifikation, die an den erfindungsgemäßen Zeolithen vorgenommen werden kann, ist die Dealuminierung, bei der ein Teil der Aluminumatome durch Silicium ersetzt wird oder die Zeolithe durch beispielsweise hydrothermale Behandlung in ihrem Aluminumgehalt abgereichert werden. An eine hydrothermale Dealuminierung schließt sich vorteilhafterweise eine Extraktion mit Säuren oder Komplexbildnern an, um gebildetes Nichtgitteraluminium zu entfernen. Der Ersatz von Aluminium durch Silicium kann beispielsweise mit Hilfe von (NH₄)₂SiF₆ oder SiCl₄ erfolgen. Beispiele für Dealuminierungen von Y-Zeolithen finden sich in Corma et al., Stud. Surf. Sci. Catal. 37 (1987), Seiten 495 bis 503.

Die Katalysatoren kann man als Stränge mit Durchmessern von z.B. 1 bis 4 mm oder als Tabletten mit z.B. 3 bis 5 mm Durchmesser für die Aminierung der Olefine einsetzen.

Aus dem beispielsweise zu Strängen verformten Katalysator kann man durch Mahlen und Sieben ein Wirbelgut in der Größe von 0,1 bis 0,8 mm erhalten.

Die Substituenten R¹, R², R³, R⁴, R⁵ und R⁶ in den Verbindungen I, II und III haben folgende Bedeutungen:
R¹,R²,R³,R⁴,R⁵,R⁶
   - Wasserstoff,
   - C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, n-Hexyl, iso-Hexyl, n-Heptyl, iso-Heptyl, n-Octyl und iso-Octyl,
   - C₂- bis C₈-Alkenyl wie Vinyl und Allyl,
   - C₂H und Propargyl,
   - C₅- bis C₈-Cycloalkyl wie Cyclopentyl, Cyclohexyl, Cycloheptyl und Cyclooctyl,
   - C₅- bis C₁₀-Alkyl-cycloalkyl,
   - C₅- bis C₁₀-Cycloalkyl-alkyl,
R¹ und R²
   - gemeinsam eine gesättigte oder ungesättigte C₃- bis C₉-Alkylendikette, bevorzugt -(CH₂)₄-, -(CH₂)₅-, -(CH₂)₇- und -CH=CH-CH=CH-,
R³ und R⁵
   - gemeinsam eine C₃- bis C₈-Alkylendikette, besonders bevorzugt -(CH₂)₃-, -(CH₂)₄-, -(CH₂)₅-, -(CH₂)₆- und -(CH₂)₇-, insbesondere -(CH₂)₃- und -(CH₂)₄-.

### Beispiele

### Katalysatorsynthese

60g SSZ-37 wurden mit 40g Boehmit und 2g Ameisensäure versetzt, im Kneter kompaktiert und unter Wasserzusatz (105 ml) 45 Minuten verknetet. In einer Strangpresse wurden mit einem Preßdruck von 40 bar 2mm Stränge erzeugt, 16 h bei 120°C getrocknet und anschließend 16 h bei 500°C calciniert.

### Aminierungsbeispiele

Die Versuche wurden in einem Rohrreaktor (6 mm Innendurchmesser) unter isothermen Bedingungen bei 260 bis 300°C und einem Druck von 280 bar mit einem Gemisch aus Isobuten und Ammoniak im molaren Verhältnis von 1:1,5 durchgeführt. Die Reaktionsprodukte wurden im Gaschromatographen analysiert.

Die Ergebnisse sind in Tabelle 1 zusammengestellt.

**Tabelle 1:**

| tert.-Butylamin (NH₃: C₄H₈ = 1,5) | | | | | |
|---|---|---|---|---|---|
| Druck | Temperatur | tert.-Butylamin-Ausbeute [Gew.-%] | | | Litergewicht |
| [bar] | [°C] | WHSV 0,7 [g/g·h] | WHSV 1,5 [g/g·h] | WHSV 3 [g/g·h] | [kg/l] |
| 280 | 260 | 17,6 | | | 0,47 |
| 280 | 270 | 20,2 | 17,0 | 13,0 | 0,47 |
| 280 | 280 | 17,7 | 16,5 | 14,0 | 0,47 |
| 280 | 300 | | | 11,5 | 0,47 |

## Patentansprüche

1. Verfahren zur Herstellung von Aminen der allgemeinen Formel I 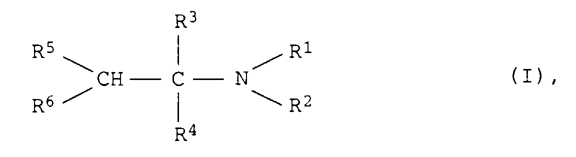 in der
R¹,R²,R³,R⁴,R⁵,R⁶ Wasserstoff, C₁- bis C₂₀-Alkyl, C₂- bis C₈-Alkenyl, C₂H, Propargyl, C₂- bis C₈-Cycloalkyl, C₂- bis C₁₀-Alkyl-cycloalkyl oder C₇- bis C₁₀-Cycloalkyl-alkyl,
R¹ und R² gemeinsam eine gesättigte oder ungesättigte C₃- bis C₉-Alkylendikette und
R³ und R⁵ gemeinsam eine C₂- bis C₈-Alkylendikette
bedeuten, durch Umsetzung von Olefinen der allgemeinen Formel II 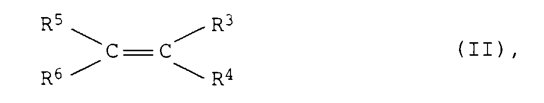 in der R³, R⁴, R⁵ und R⁶ die oben genannten Bedeutungen haben, mit Ammoniak oder primären oder sekundären Aminen der allgemeinen Formel III 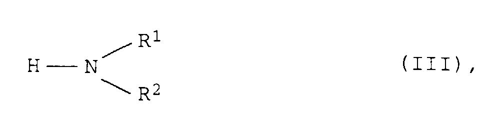 in der R¹ und R² die oben genannten Bedeutungen haben, bei Temperaturen von 200 bis 350°C und Drücken von 100 bis 300 bar in Gegenwart eines Heterogenkatalysators, dadurch gekennzeichnet, daß man als Heterogenkatalysator Zeolithe des Typs SSZ-37 einsetzt.

2. Verfahren zur Herstellung von Aminen I nach Anspruch 1, dadurch gekennzeichnet, daß man das gebildete Amin I abtrennt und die nichtumgesetzten Einsatzstoffe II und III zurückführt.

3. Verfahren zur Herstellung von Aminen nach den Ansprüchen 1 bis 2, dadurch gekennzeichnet, daß man als Olefin II Isobuten, Diisobuten, Cyclopenten, Cyclohexen oder Polyisobuten einsetzt.

4. Verfahren zur Herstellung von Aminen nach den Ansprüchen 1 bis 3, dadurch gekennzeichnet, daß man als Heterogenkatalysatoren SSZ-37 Zeolithe in der H-Form einsetzt.

5. Verfahren zur Herstellung von Aminen nach den Ansprüchen 1 bis 4, dadurch gekennzeichnet, daß man als Heterogenkatalysatoren SSZ-37 Zeolithe, die mit einer Säure, insbesondere einer aus der Gruppe Salzsäure, Flußsäure, Schwefelsäure, Oxalsäure, Phosphorsäure oder deren Gemischen, behandelt sind, einsetzt.

6. Verfahren zur Herstellung von Aminen nach den Ansprüchen 1 bis 5, dadurch gekennzeichnet, daß man als Heterogenkatalysatoren SSZ-37 Zeolithe, die mit einem oder mehreren Übergangsmetallen dotiert sind, einsetzt.

7. Verfahren zur Herstellung von Aminen nach den Ansprüchen 1 bis 6, dadurch gekennzeichnet, daß man als Heterogenkatalysatoren SSZ-37 Zeolithe, die mit einem oder mehreren Elementen der Seltenen Erden dotiert sind, einsetzt.

8. Verfahren zur Herstellung von Aminen nach den Ansprüchen 1 bis 7, dadurch gekennzeichnet, daß man als Heterogenkatalysatoren SSZ-37 Zeolithe in der Ammoniumform einsetzt.

9. Verfahren zur Herstellung von Aminen nach den Ansprüchen 1 bis 8, dadurch gekennzeichnet, daß man als Heterogenkatalysatoren SSZ-37 Zeolithe einsetzt, die mit einem oder mehreren Elementen aus der Gruppe der Alkali-, Erdalkalimetalle oder Erdmetalle dotiert sind.

10. Verfahren zur Herstellung von Aminen nach den Ansprüchen 1 bis 9, dadurch gekennzeichnet, daß man als Heterogenkatalysatoren SSZ-37 Zeolithe einsetzt, die mit einem Bindemittel verformt und bei Temperaturen von 200 bis 600°C calciniert sind.

11. Verfahren zur Herstellung von Aminen nach den Ansprüchen 1 bis 10, dadurch gekennzeichnet, daß man als Heterogenkatalysatoren dealuminierte SSZ-37 Zeolithe einsetzt.

## Claims

1. A process for preparing amines of the general formula I 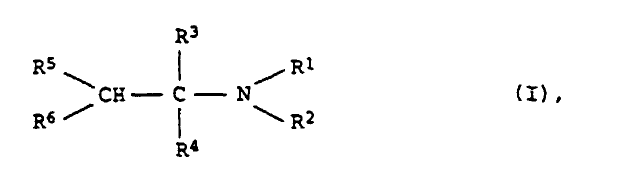 where
R¹,R²,R³,R⁴,R⁵,R⁶ are each hydrogen, C₁-C₈-alkyl, C₂-C₈-alkenyl, C₂H, propargyl, C₅-C₈-cycloalkyl, C₅-C₁₀-alkylcycloalkyl or C₅-C₁₀-cycloalkylalkyl,
R¹ and R² are together a saturated or unsaturated C₃-C₉-alkylene dichain, and
R³ and R⁵ are together a C₃-C₈-alkylene dichain,
by reacting olefins of the general formula II 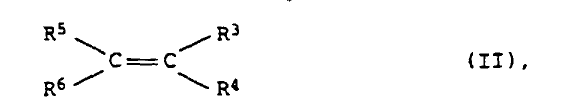 where R³, R⁴, R⁵ and R⁶ are each as defined above, with ammonia or primary or secondary amines of the general formula III 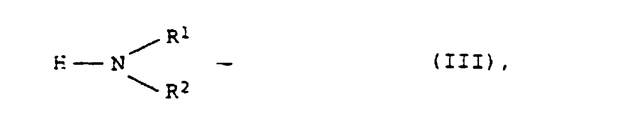 where R¹ and R² are each as defined above, at temperatures from 200 to 350°C and pressures from 100 to 300 bar in the presence of a heterogeneous catalyst, which comprises using a heterogeneous catalyst comprising zeolites of the type SSZ-37.

2. A process for preparing amines I as claimed in claim 1, wherein the product amine I is separated off and the unconverted feed materials II and III are recycled.

3. A process for preparing amines as claimed in claim 1 or 2, wherein the olefin II used is isobutene, diisobutene, cyclopentene, cyclohexene or polyisobutene.

4. A process for preparing amines as claimed in any of claims 1 to 3, wherein the heterogeneous catalyst used is an SSZ-37 zeolite in the H-form.

5. A process for preparing amines as claimed in any of claims 1 to 4, wherein the heterogeneous catalyst used is an SSZ-37 zeolite which has been treated with an acid, in particular with an acid selected from the group consisting of hydrochloric acid, hydrofluoric acid, sulfuric acid, oxalic acid, phosphoric acid and mixtures thereof.

6. A process for preparing amines as claimed in any of claims 1 to 5, wherein the heterogeneous catalyst used is an SSZ-37 zeolite doped with one or more transition metals.

7. A process for preparing amines as claimed in any of claims 1 to 6, wherein the heterogeneous catalyst used is an SSZ-37 zeolite doped with one or more rare earth elements.

8. A process for preparing amines as claimed in any of claims 1 to 7, wherein the heterogeneous catalyst used is an SSZ-37 zeolite in the ammonium form.

9. A process for preparing amines as claimed in any of claims 1 to 8, wherein the heterogeneous catalyst used is an SSZ-37 zeolite doped with one or more elements from the group consisting of alkali, alkaline earth or earth metals.

10. A process for preparing amines as claimed in any of claims 1 to 9, wherein the heterogeneous catalyst used is an SSZ-37 zeolite molded with a binder and calcined at temperatures from 200 to 600°C.

11. A process for preparing amines as claimed in any of claims 1 to 10, wherein the heterogeneous catalyst used is a dealuminated SSZ-37 zeolite.

## Revendications

1. Procédé de préparation d'amines de la formule générale I 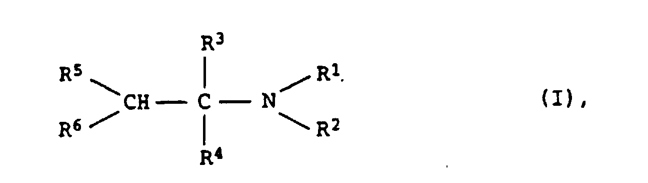 dans laquelle
R¹, R², R³, R⁴, R⁵, R⁶ représentent l'hydrogène, un radical alkyle en C₁-C₈, alcényle en C₂-C₈, C₂H, propargyle, cycloalkyle en C₅-C₈, alkylcycloalkyle en C₅-C₁₀ ou cycloalkylalkyle en C₅-C₁₀,
R¹ et R² représentent ensemble une double chaîne alkylène en C₃-C₉ saturée ou insaturée et
R³ et R⁵ représentent ensemble une double chaîne alkylénique en C₃-C₈, par réaction d'oléfines de la formule générale II 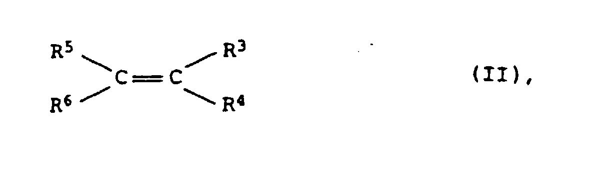
dans laquelle R³, R⁴, R⁵ et R⁶ ont les significations ci-dessus, avec de l'ammoniac ou des amines primaires ou secondaires de la formule générale III 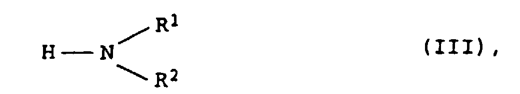 dans laquelle R¹ et R² ont les significations ci-dessus, à des températures de 200 à 350C° et sous des pressions de 100 à 300 bar, en présence d'un catalyseur hétérogène, caractérisé en ce que l'on utilise comme catalyseur hétérogène des zéolithes de type SSZ-37.

2. Procédé de préparation d'amines I selon la revendication 1, caractérisé en ce que l'on sépare l'amine I formée et que l'on recycle les composés II et II de départ non convertis.

3. Procédé de préparation d'amines selon les revendications 1 à 2, caractérisé en ce que l'on met en oeuvre, en tant qu'oléfines II, de l'isobutène, du diisobutène, du cyclopentène, du cyclohexène ou du polyisobutène.

4. Procédé de préparation d'amines selon les revendications 1 à 3, caractérisé en ce que l'on met en oeuvre, en tant que catalyseur hétérogène, des zéolithes de type SSZ-37 sous leur forme H.

5. Procédé de préparation d'amines selon les revendications 1 à 4, caractérisé en ce que l'on met en oeuvre, en tant que catalyseur hétérogène, des zéolithes SSZ-37 traitées par un acide, en particulier un acide choisi dans le groupe formé par l'acide chlorhydrique, l'acide fluorhydrique, l'acide sulfurique, l'acide oxalique, l'acide phosphorique ou leurs mélanges.

6. Procédé de préparation d'amines selon les revendications 1 à 5, caractérisé en ce que l'on met en oeuvre, en tant que catalyseur hétérogène, des zéolithes SSZ-37 dopées au moyen d'un ou plusieurs métaux de transition.

7. Procédé de préparation d'amines selon les revendications 1 à 6, caractérisé en ce que l'on met en oeuvre, en tant que catalyseur hétérogène, des zéolithes SSZ-37 dopées au moyen d'un ou plusieurs éléments parmi les terres rares.

8. Procédé de préparation d'amines selon les revendications 1 à 7, caractérisé en ce que l'on met en oeuvre, en tant que catalyseur hétérogène, des zéolithes SSZ-37 sous la forme ammoniacale.

9. Procédé de préparation d'amines selon les revendications 1 à 8, caractérisé en ce que l'on met en oeuvre, en tant que catalyseur hétérogène, des zéolithes SSZ-37 dopées au moyen d'un ou plusieurs éléments du groupe des métaux alcalins, alcalino-terreux ou terreux.

10. Procédé de préparation d'amines selon les revendications 1 à 9, caractérisé en ce que l'on met en oeuvre, en tant que catalyseur hétérogène, des zéolithes SSZ-37 façonnées avec un liant et calcinées à des températures de 200 à 600°C.

11. Procédé de préparation d'amines selon les revendications 1 à 10, caractérisé en ce que l'on met en oeuvre, en tant que catalyseur hétérogène, des zéolithes SSZ-37 désaluminées.
